# EUROPEAN PATENT SPECIFICATION

(11) **EP 4 491 209 B1**
(45) Date of publication and mention of the grant of the patent: **11.02.2026**
(21) Application number: 23185330.0
(22) Date of filing: 13.07.2023
(51) Int. Cl.: A61M 11/06, A61M 11/00

(54) **JET NEBULIZER AND AEROSOLIZING METHOD**
STRAHLVERNEBLER UND AEROSOLIERUNGSVERFAHREN
NÉBULISEUR À JET ET PROCÉDÉ D'AÉROSOLISATION

(43) Date of publication of application: 15.01.2025
(73) Proprietor: Melticare AB, 622 75 Visby (SE)
(72) Inventor: Erenbrand, Niclas, 622 75 Visby (SE); Rüdén, Roger, 116 33 Stockholm (SE)
(74) Representative: AWA Sweden AB

(56) References cited:
- EP-A2- 0 276 939
- US-A- 4 746 067
- US-A1- 2013 037 020
- US-A1- 2014 373 831

## Description

### TECHNICAL FIELD

The inventive concept relates to the field of nebulizers, and more specifically to pneumatic medical nebulizers, also referred to as jet-type nebulizers. There is disclosed an improved jet nebulizer, and an improved aerosolizing method.

### BACKGROUND

A nebulizer is a relatively small device arranged to aerosolize or atomize a liquid medication into a fine spray or mist, for delivery to the lower respiratory tract of a patient via inhalation. The liquid is said to be nebulized or aerosolized. The particles or droplets may be in the range of 1-5 µm. Uses include for instance hospital use, pre-hospital use (ambulances), military use, and rescue services.

Nebulizers and other mist generators for therapeutic use exist in a wide variety. Therapeutic use includes delivery of aerosolized medications to the lungs and air passageways of a patient. A nebulizer may be used for a variety of chronic or acute conditions, for example respiratory illnesses as asthma and bronchiectasis. Nebulizers may also be used for diagnostic purposes, e.g. by using aerosolized radioactive isotopes for image generation. The mist may be administered to a patient via a face mask or a mouthpiece attached to the nebulizer.

The present inventive concept relates to jet nebulizers, also referred to as pneumatic nebulizers. The following description of the general structure and operation of jet nebulizers applies to the inventive nebulizer. In a jet nebulizer, compressed or pressurized gas, e.g. compressed air or pressurized oxygen gas, is used as the driving force for producing the aerosol or mist. Typically, a hose connects a source of pressurized gas to the nebulizer. A liquid medication dose is present in a liquid reservoir or container of the nebulizer. The pressurized gas is introduced into a gas inlet of a gas channel, which typically extends centrally in the nebulizer along a center axis of the nebulizer from the gas inlet located at a base of the nebulizer, to a gas outlet located internally and centrally in the nebulizer. The pressurized gas exits at high speed at the gas outlet. In response thereto, liquid present in the liquid container is aspired or drawn into a liquid passageway by venturi effect. The outlet of the liquid passageway is located adjacent to the gas outlet of the gas channel, together forming a nozzle for generating a liquid/gas mixture. A jet nebulizer nozzle may be of internal or external type. In an internal-type nebulizer nozzle, the gas flow interacts with the liquid prior to leaving the nozzle. In an external-type nebulizer nozzle, the gas flow and the liquid interacts not until after leaving the nozzle.

A jet nebulizer further comprises a baffle or impact surface (also termed target surface) located adjacent and spaced from the nozzle. The baffle is located in the liquid/gas stream generated by the nozzle for producing an aerosol of smaller particles. Larger particles are returned to the liquid reservoir. The produced aerosol flow is then directed to an outlet of the nebulizer in order to be administered to the patient.

Producing an ideal mist or aerosol requires consideration of several factors. Particle size and particle distribution are important factors. Another factor is uniformity: The aerosol should be delivered to the lungs and lower respiratory tracts in a uniform manner, and with a substantially uniform particle size distribution.

While existing nebulizers may be effective in some situations, there are room for improvements. Present jet nebulizers present one or more of the following shortcomings:
No or limited function in tilted orientations of the nebulizer, resulting in no or low delivery of the medicament.
Liquid leakage in tilted orientations of the nebulizer.
Non-optimal particle size in the generated aerosol, especially in tilted orientations.

EP276939 discloses a nebulizer for introducing a liquid in fine droplet form into a gas flow, including a reservoir for the liquid, a side wall, a base wall of generally conical shape, the base wall extending from a lower outer periphery adjacent said side wall to a central raised part, the base wall being covered by a flange plate whereby there is provided a capillary path from the lower outer periphery to a centrally mounted jet.

US2014/0373831 discloses a pre-filled, single-use, disposable medication chamber for use in a medical nebulizer system that delivers a mist of properly sized aerosol particles of medicament to the patient. The nebulizer can be effectively used in conjunction with conventional nebulizer cap, tee and mouthpiece devices that interface with the patient.

US2013/0037020 discloses a nebulizer kit includes a case body, a flow channel formation member that covers an opening of the case body, an outside air introduction tube erected vertically from a base surface of the flow channel formation member and including a long groove formed in an inner circumferential surface thereof, a compressed air introduction tube below the outside air introduction tube within the case body and extending toward the interior of the outside air introduction tube, and an atomizing area formation member.

US4746067 discloses A device and method for aerosolizing a liquid in a gas using a pressurized gas by creating the fog or "steam" at atmospheric pressure and room temperature. The liquid is drawn into the gas stream using a venturi pipe. The amount of entrained liquid is enhanced by directing the flow of liquid and gas against a deflector. Liquid entrainment is further enhanced by directing the flow past projections causing turbulence in the stream.

### SUMMARY OF INVENTION

In the light of the above, it is an object of the present inventive concept to provide an improved jet nebulizer, and an improved aerosolizing method which remedies or at least reduces one or more of the disadvantages of known solutions.

The invention is defined by the appended claims.

According to a first aspect of the inventive concept, there is provided a jet nebulizer for creating an aerosol from a liquid, such as a liquid medication, comprising:
a tubular housing having a sidewall which extends circumferentially about a main axis of the nebulizer;
a conical structure having a concave conical surface, which extends between a cone top and a cone base, which together with the sidewall of the tubular housing forms an inner volume in the nebulizer; and
an aerosol generating structure which is arranged to generate an initial aerosol flow from said liquid and to guide the initial aerosol flow along the concave conical surface towards the cone base,
wherein a base wall portion of the sidewall extends from an axial level of the cone base, with respect to the main axis, to an axial level between the cone top and the cone base where the base wall portion transits into an inclined wall portion of the sidewall, said inclined wall portion being inclined in relation to the base wall portion inwardly towards said main axis; and
wherein the sidewall is arranged to redirect the initial aerosol flow guided along the concave conical surface to form a reverse aerosol flow in the inner volume.

### Terminology

In the present disclosure, directional terms such as downwardly, upwardly, top, base, and the like should be interpreted as directions of the nebulizer when the latter assumes an upright orientation with its main axis vertically oriented.

In the present disclosure, the geometrical main axis of the nebulizer is used as a reference for geometrical terms such as circumferential, peripheral, axial level, and radially. For instance, the cone top and the cone base are located at mutually different axial levels with respect to the main axis.

In the present disclose, a "tilted orientation" of the nebulizer refers to an inclined orientation in which the main axis of the nebulizer forms an angle of up to and including 90 degrees from the vertical. Thus, a tilted orientation may be a horizontal orientation (90 degrees) of the main axis, or an angled orientation of the main axis between vertical and horizontal (< 90 degrees).

With reference to the main axis of the inventive nebulizer, the initially generated aerosol is guided along the concave conical surface of the conical structure, from the cone top towards the cone base. Due to the concave shape of the flow guiding conical surface, the initial downwardly directed aerosol flow will thus follow the concave curved shape of the conical structure. At a lower position of the inner volume, closer to the cone base, the aerosol flow direction is reversed (turned) into an upwardly directed reverse aerosol flow. It will be appreciated that this redirection will occur at an axial level essentially in level with the actual and changing liquid surface. Thus, the redirection level will become continuously closer to the cone base as the operation continues as the liquid is withdrawn from the inner volume. The reverse aerosol flow is guided upwardly by the sidewall of the tubular housing. Due to the inventive design of the sidewall, where the sidewall presents a change in inclination gradient at a level between above the cone base, the inner volume will present aa larger cross section at the level where the flow redirection occurs. Especially, the combined effect of a concave conical guiding surface and the design of the sidewall will create an advantageous larger volume for the aerosol flow, for the reversal of the flow direction, and for the vortex formation. In terms of performance of the nebulizer, the inventors have confirmed that the resulting reversal of the aerosol flow generates a final aerosol output, which is consistent and has excellent and stable parameter values. The inventors has also demonstrated that the inventive design of the inner volume, with an increased cross section where the redirection or flow reversal takes place, results in an advantageous vortex formation of the aerosol flow, which further enhances the performance parameters. These advantages will be explained in more detail later.

A specific advantage of the inventive nebulizer is that the flow path described above, and the resulting aerosol flow output from the nebulizer, is essentially the same in a tilted orientation. For instance, in a tilted orientation where the main axis of the nebulizer is in a horizontal plane, the flow path, the flow reversal, and the vortex formation will be essentially the same as in the upright orientation.

A second advantage of the inventive nebulizer is that the inclined wall portion of the inner wall creates a liquid barrier for the liquid in a tilted orientation, preventing the liquid from escaping through the outlet of the nebulizer. This will prevent liquid leakage, and will ensure that a correct aerosol dose is distributed to the patient. In inclined orientations, the inclined wall portion also acts as a downhill slope guiding the liquid towards the liquid inlet of the aerosol generating structure. The inclined wall portion starts at an axial level above the cone base. Thus, in a tilted orientation of the nebulizer, the liquid will be collected and retained at least at a location above the lowest part of the base wall portion. By arranging the liquid inlet at the cone base at the base wall portion it is ensured that liquid is present and available at the liquid inlet also in a tilted orientation. Depending on the design of the inventive nebulizer, the inclined wall portion may also have the function of guiding the reverse aerosol flow towards an outlet of the nebulizer.

In some embodiments, the nebulizer is structured and arranged to allow adjustment and/or pre-adjustment of a baffle distance between a mist generating nozzle and an impact surface of a baffle. An adjustable baffle distance allows for a suitable particle size distribution to be set depending on different gas flow rates. For a given gas flow rate, a suitable aerosol mist may be generated for optimized treatment. Especially, it is possible to set the "dominating" portion of the particle size in order obtain an optimal treatment effect for various disease conditions.

An adjustable baffle distance may be implemented by arranging the baffle as part of a top part of the nebulizer, and by connecting the top part supporting the baffle to the rest of the nebulizer via an adjustable connection, such as a threaded connection. The baffle distance may then be increased or decreased by rotating the top part in the suitable direction. The adjustment may be stepless, or stepwise with fixed positions for different treatments.

The sidewall of the housing may optionally further comprise a curved wall portion, which forms a curved transition of the sidewall between the base wall portion and the inclined wall portion, wherein said curved wall portion presents a concave inner surface facing the inner volume. In such embodiments, concave inner surface of the curved wall portion may contribute to an efficient guiding of the reverse aerosol flow.

In preferred embodiments, the inner volume may present its largest cross section, with respect to said main axis, at the cone base. Such embodiments have the advantage that the liquid in the inner volume will be present at the cone base also in tilted orientations of the nebulizer. Expressed in other words, in preferred embodiments the sidewall of the housing is designed such that, when the nebulizer is in a 90 degree tilted orientation with the main axis horizontally directed, then the liquid present in the inner volume is collected at the lowest part of the sidewall, and will be deepest adjacent the cone base. Since the liquid is preferably drawn in from the inner volume adjacent the cone base, this design ensures that essentially all of the liquid present in the inner volume can be drawn in both in the upright orientation and in the tilted orientation.

The conical structure may optionally comprise a central compressed air passageway, and a liquid passageway which is arranged peripherally of the compressed air passageway and which has a liquid inlet located at the sidewall of the tubular housing, preferably close to or adjacent the cone base. The liquid inlet may be formed by a continuous circumferential gap or play between the cone base and the sidewall. In alternative embodiments, the liquid inlet may be formed by a plurality separate openings, notches, or the like formed in the cone base. In general, at least part of the liquid inlet is preferably be located at the deepest or lowest part of the liquid when the nebulizer is in a tilted orientation, in order to ensure that most of the liquid is drawn into the liquid passageway.

In order to avoid liquid being guided away from the liquid inlet in a horizontal orientation of the nebulizer, the base wall portion of the sidewall preferably extends in a direction with respect to the main axis such that the sidewall does not diverge outwardly from the main axis. In preferred embodiments, the base wall portion extends in a direction parallel to the main axis of the nebulizer. This ensures that the base wall portion will guide the liquid towards the cone base and a liquid inlet in tilted orientations of the nebulizer up to 90 degrees.

In preferred embodiments, the nebulizer further comprises an internal tubular structure which projects into the inner volume and which has an opening located at an axial level, with respect to the main axis, between the cone top and the cone base, wherein said internal tubular structure together with a portion of the sidewall forms a cavity arranged to act as a liquid trap or liquid pocket for the liquid in a tilted or inverted orientation of the nebulizer. In the upright orientation of the nebulizer, the internal tubular structure projects downwardly into the inner volume of the nebulizer. The liquid trap is formed between the tubular structure and the sidewall of the housing, preferably between the tubular structure and the inclined wall portion of the housing. Thereby, the liquid trap is formed as a circumferentially extending, ring-shaped cavity, which is open downwardly and which tapers and is closed upwardly.

The volume of the liquid trap is preferably selected with respect to the intended liquid fill volume, e.g. 5 ml, such that the liquid trap can hold the entire liquid fill volume. The liquid trap has the advantage that it prevents the liquid from exiting the nebulizer in any orientation of the nebulizer, including upside down orientations, provided that the liquid volume does not exceed the volume of the liquid trap. No matter how the nebulizer is oriented in space, the liquid cannot escape from the nebulizer, ensuring that the correct dose is eventually administered to the patient when the nebulizer is oriented with a tilt angle of 0-90 degrees. In a tilted orientation of the nebulizer, e.g. in a horizontal orientation, part of the liquid may be present inside the lower portion of the liquid trap. The liquid surface will still be well below the aerosol generating structure, and the nebulizer is fully operational in a tilted horizontal orientation. Especially, the inventive flow reversal concept described above is present also in the tilted orientation.

In embodiments including such an internal tubular structure forming a liquid trap, the nebulizer has thus both the advantage of being fully operational in tilted orientations up to 90 degrees, and the additional advantage of keeping the liquid inside the nebulizer also for tilt angles over 90 degrees.

When the nebulizer is in its upright orientation and in operation, the liquid trap cavity does not contain any liquid. Due to the aerosol flow inside the inner volume, especially the reverse aerosol flow, an overpressure is created inside the cavity which will prevent or at least restrict reverse aerosol flow into the cavity.

During operation, some liquid particles in the aerosol flow may adhere to the internal tubular structure and eventually fall off from the lower edge of the tubular structure as droplets being returned to the liquid present in the inner volume.

In embodiments with an internal tubular structure as defined above, the internal tubular structure may optionally present an outwardly flaring skirt portion defining the lower opening of the internal tubular structure. This design has the advantage that the reverse aerosol flow is efficiently directed by the skirt into the lower opening of the internal tubular structure, both in the upright orientation and in the tilted orientation of the nebulizer. The skirt may have an angle and curvature corresponding to the angle and curvature of the sidewall of the housing.

In some embodiments including an internal tubular structure as described above, the nebulizer may comprise a second liquid trap, which has a smaller volume than the above-mentioned first liquid trap and which is located axially higher up in the nebulizer. Such embodiments may be preferred in order to avoid a too large size of the first liquid trap, which may disturb the aerosol flow and the vortex formation inside the inner volume.

According to the inventive concept, the aerosol generating structure is arranged to guide the initial aerosol flow along the concave conical surface. Such a flow guiding may be implemented by providing the baffle with a flow guide, especially a circumferentially extending flow guide. In some embodiments, the baffle may be a circular plate provided with a downwardly projecting flow guide skirt extending circumferentially around the plate. The flow guide may preferably have an inclined flow guiding surface.

According to a second aspect of the inventive concept, there is provided a method for generating an aerosol flow in a jet nebulizer, said method comprising:
generating an initial aerosol inside a nebulizer from a liquid and a pressurized gas;
guiding the initial aerosol as a primary aerosol flow along a concave conical surface from a top of the concave conical surface towards a base of the concave conical surface, said concave conical surface together with a sidewall of the nebulizer defining an inner volume of the nebulizer;
at the base of the concave conical surface, redirecting the aerosol flow by the sidewall to form a reverse aerosol flow; and
guiding the reverse aerosol through the inner volume to an outlet of the nebulizer.

As in the inventive nebulizer according to the first aspect described above, the inventive method may further comprise using said primary aerosol flow and said reverse aerosol flow in combination to form an aerosol vortex within said inner volume, said vortex contributing to the direction and speed of the reverse upward flow, and contributing to an increased degree of particle collisions, i.e. a reduced particle size.

The inventive method for aerosol formation may optionally be used in combination with the design of the sidewall of the housing as described above, in order to allow the method to be operational in a tilted orientation of the nebulizer.

Further optional features of the inventive method are set out in the dependent claims.

The inner volume of the nebulizer have multiple functions. A first function of the inner volume is to act as a liquid reservoir for the liquid to be aerosolized. The initial liquid fill volume may vary depending on recommended patient dose, and could as an example be around 4 to 5 ml. The initial liquid fill volume will typically be less than the total volume of the inner volume, such that the liquid is present only at a lower part of the inner volume. The location of the lower part of the inner volume will vary depending on the orientation of the nebulizer, e.g. an upright orientation or a tilted orientation. A second function of the inner volume is to create a space for the inventive aerosol flow pattern and the vortex formation. During operation, the initial aerosol flow will be directed along the concave conical surface towards the cone base. This flow takes place in the inner volume. The aerosol flow is redirected or reversed by the sidewall of the tubular housing to form a reverse aerosol flow also present in the inner volume. It will be appreciated that the liquid filled portion of the inner volume will gradually decrease during operation of the nebulizer.

### BRIEF DESCRIPTION OF THE DRAWINGS

The inventive concept, a non-limiting embodiment, and further advantages of the inventive concept will now be described with reference to the drawings in which:
- Fig. 1: is a cross-sectional view of an embodiment of a nebulizer.
- Fig. 2: is an exploded cross-sectional view illustrating parts of the nebulizer.
- Fig. 3: is an exploded perspective view illustrating parts of the nebulizer.
- Fig. 4: is a perspective view, partly in cross-section, of the nebulizer.
- Figs 5a and 5b: schematically illustrate an adjustment function of the nebulizer.
- Figs 6a to 6c: schematically illustrate an internal aerosol flow in the nebulizer.
- Fig. 7: illustrates in enlarged scale liquid being drawn into a liquid passageway.
- Figs 8a and 8b: illustrate the operation of the nebulizer in an upright orientation and in a tilted orientation, respectively.

### DETAILED DESCRIPTION OF PREFERRED EMBODIMENTS

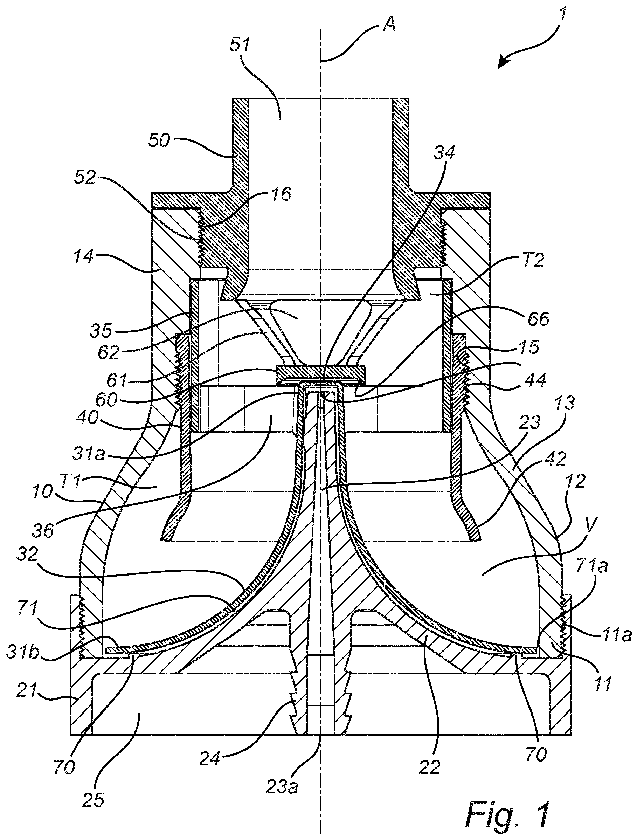

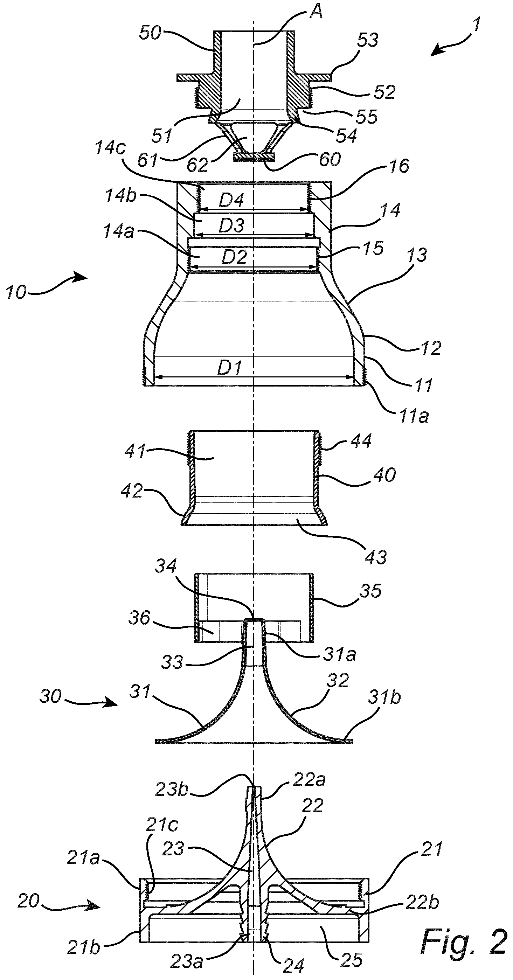

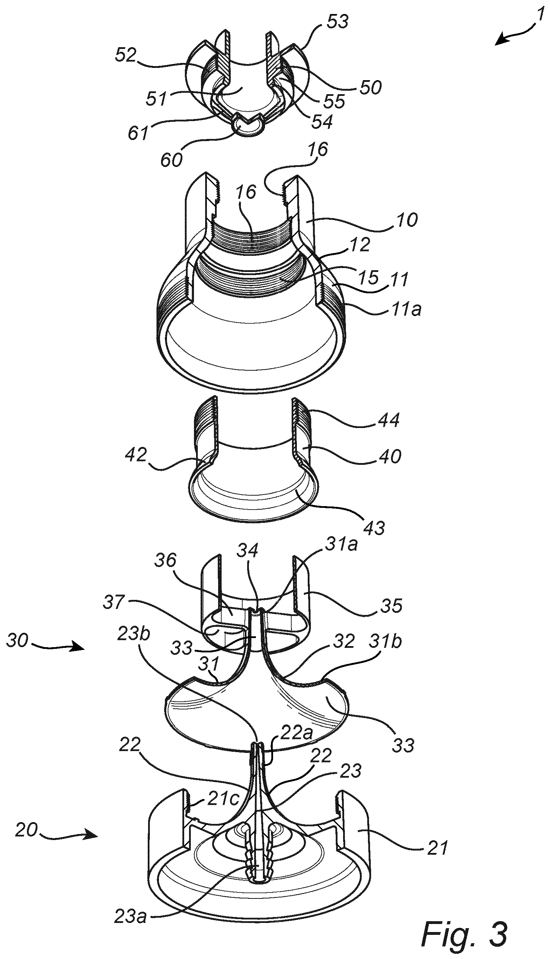

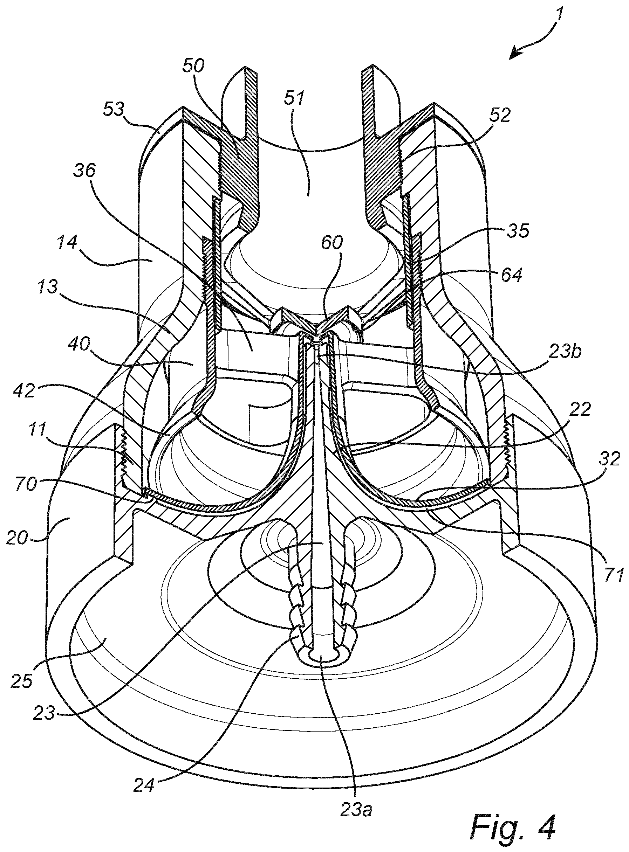

Reference is now made to Figs 1 and 4, illustrating an embodiment of a nebulizer 1, and Figs 2 and 3 illustrating the separate parts of the nebulizer 1. As best shown in the exploded views in Figs 2 and 3, the nebulizer 1 comprises a tubular housing 10, a bottom part 20, an insert part 30, a tubular part 40, and a top part 50. These parts of the nebulizer 1 can be made, e.g. molded, from a rigid plastic material. They may also be made from a metal, such as aluminum, or from a combination of plastic material and metal. The parts may be manufactured as shown in Figs 2 and 3 as a plurality of distinct components to be assembled into the final nebulizer 1. In some embodiments, the nebulizer parts can be at least partly separated for cleaning if the nebulizer is designed for re-use. In alternative embodiments, one or more of these parts can be manufactured in a single integrated piece. The nebulizer 1 may also be designed for single use.

The overall design of the nebulizer 1 is preferably circular symmetric with respect to a main axis A of the nebulizer 1. In Fig. 1, the nebulizer 1 is shown in its upright orientation with its main axis A vertically directed. With respect to dimensions and as a non-limiting example, the axial height of the nebulizer 1 may be in the order of 70 mm, and the largest cross-section may be in the order of 50 mm. In general, the outer dimensions of the nebulizer 1 should preferably be such that the nebulizer 1 can be held and oriented with one hand.

The tubular housing 10 has a specifically designed sidewall shape to provide specific functions and advantages of the nebulizer 1 as will be described later. In very schematic terms, one could say that, in preferred embodiments as the one illustrated in Fig. 2, the tubular housing 10 has an overall outer shape resembling an elephant foot, i.e. an upper cylindrical part of a smaller diameter which transits via an inclined part into a lower cylindrical part of a larger diameter. The tubular housing 10 is formed by a sidewall having a varying inner diameter along the main axis A. In order from its lower end or base end, the sidewall comprises a base wall portion 11, a curved transition wall portion 12, an inclined wall portion 13, and a top wall portion 14.

The base wall portion 11 of the sidewall extends from a lower end of the sidewall to an axial level, with respect to the main axis A, where the base wall portion 11 transits, via the curved wall portion 12, into the inclined wall portion 13. In the illustrated embodiment, the base wall portion 11 is oriented in parallel with the main axis A, i.e. it extends vertically in the illustrated upright orientation of the nebulizer 1. It will be noted that such a non-angled orientation of the lowest portion 12 of a nebulizer sidewall differs essentially in structure and function from most conventional prior-art jet nebulizers having a frusto-conical bottom wall tapering radially inwardly towards the liquid inlet, to form a water collecting funnel in the upright position. The base wall portion 11 has an inner diameter D1, which preferably is the largest inner diameter of the housing 10. The base wall portion 11 presents an outer thread 11a for connecting the bottom part 20 to the housing 10.

The curved transition wall portion 12 of the housing sidewall forms a transition between the base wall portion 10 and the inclined wall portion 13. In alternative embodiments, the transition wall portion 12 may be shorter, and it may also be dispensed with, such that the transition instead has the form of a distinct transition edge. However, the inventors have found and demonstrated that a concave curved inside of the transition wall portion 12 contributes to an enhanced aerosol flow guiding, and enhanced aerosol properties.

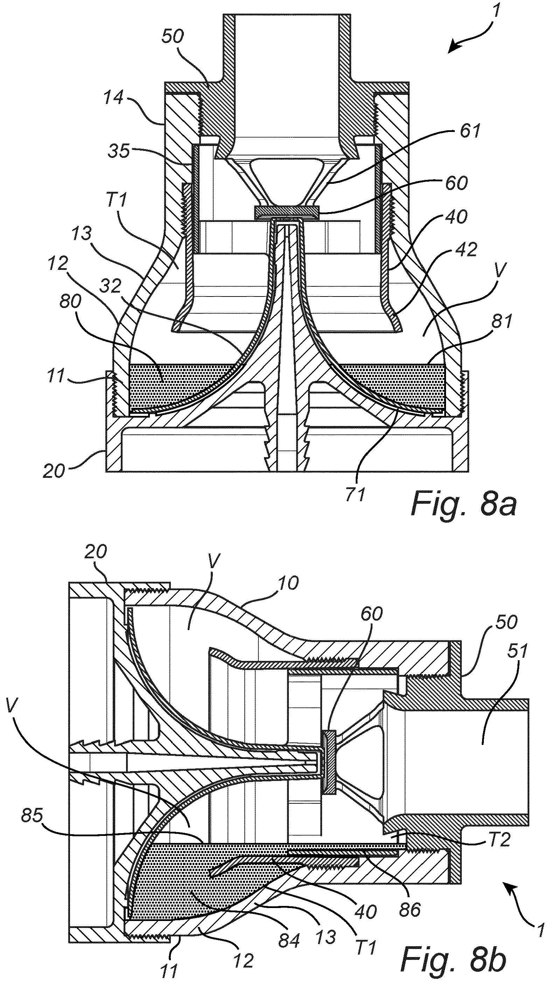

The inclined wall portion 13 of the sidewall is inclined in relation to the base wall portion 11, and it is inclined in a direction inwardly towards the main axis A. It will be appreciated that in a horizontal tilted orientation of the nebulizer 1 as the horizontal orientation shown in Fig. 8b, the lower part of the inclined wall portion 12 will be inclined upwardly as will be discussed later. In the illustrated embodiment, the inclined wall portion 13 is essentially linear. In other embodiments, it may be curved or partly curved.

In the illustrated embodiment, the top wall portion 14 of the sidewall is also extending in parallel with the main axis A. The top wall portion 14 is axially divided into three sections: a lower section 14a which transits into the inclined wall portion 13, a middle section 14b, and an upper section 14c. The three sections 14a, 14b and 14c of the top wall portion 14 have a stepwise decreasing inner diameter D2, D3, and D4, respectively, where D1 is larger than any of D2-D4. The lower section 14a has an inner thread 15 for connecting the tubular part 40 to the housing 10. The upper section 14c has an inner thread 16 for connecting the top part 50 to the housing 10. In alternative embodiments where some of the other parts are dismissed with, the upper section 14 could have a non-stepped inner side surface.

In the illustrated embodiment, the bottom part 20 of the nebulizer 1 is made as a one-piece unit arranged to be attached to the outer thread 11a on the base wall portion 11 of the tubular housing 10. The bottom part 20 comprises a cylindrical base 21 and a concave cone 22, referred to as "the lower cone 22" in the following. The cylindrical base 21 and the lower cone 22 are circular symmetric with respect to the axis A. The lower cone 22 extends from a cone top 22a to a cone base 22b. The cylindrical base 21 has an axial upper part 21a located axially above the cone base 22b, and an axial lower part 21b located axially below the cone base 22b. The cone base 22b of the lower cone 22 is integrally formed with the cylindrical base 21. The upper part 21a of the cylindrical base 21 is provided with an inner thread 21c for connecting the bottom part 20 to the housing 10 at the outer thread 11a. The bottom part 20 is manufactured as a separate part from the housing 10 in order to allow insertion of the insert part 30 and the tubular part 40 into the housing 10.

An axially directed pressurized gas channel 23 is formed inside the lower cone 22. In preferred embodiments as the one illustrated, the gas channel 23 is shaped as a jet channel having a gradually decreasing diameter from a gas inlet 23a located below the lower cone 22, to a gas outlet 23b located at the cone top 22a. A protruding attachment structure 24 for a gas hose or similar (not shown) is arranged at the gas inlet 23a. In the illustrated embodiment, the attachment structure 24 is located in its entirety inside a bottom cavity 25 formed by the lower part 21b, such that the nebulizer 1 can be placed on a table or the like in its illustrated upright position, e.g. during filling of liquid into the nebulizer 1. Preferably, the attachment structure 24 is formed as a male-type hose nipple.

For practical reasons, the insert part 30 of the nebulizer 1 is normally manufactured as a part separate from the other parts. Referring to Fig. 2, the insert part 30 comprises a conical cone 31, referred to as "the upper cone 31" in the following, having a cone top 31a and a cone base 31b. The upper cone 31 presents an upper concave conical surface 32 extending from the cone top 31a to the cone base 31b. The upper cone 31 defines an inner conical space 33 for receiving the lower cone 22. Preferably, the curvature of the upper cone 32 corresponds to the curvature of the lower cone 22. The upper cone 32 is closed at its cone top 31a by a top wall provided with a nozzle orifice 34 (Fig. 3).

As best seen in Fig. 3, the insert part 30 further comprises a stabilizing support cylinder 35. The stabilizing support cylinder 35 is arranged to be received in the middle section 14b of the top wall portion 14 of the housing 10. The stabilizing support cylinder 35 has an outer diameter D3 corresponding to the inner diameter D3 of the middle section 14b. In some embodiments, the support cylinder 35 may be detachably received in the middle section 14 of the housing 10. In some embodiments, the two parts 35 and 10 can be connected to each other by a light press fitting, preferably in a detachable manner Due to the stepwise change of the inner diameter of the upper wall portion 14 of the housing 10, the stabilizing support cylinder 35 can be positioned in a correct axial position in relation to the housing 10. The purpose of the stabilizing support cylinder 35 is to support the upper cone 31, and to stabilize the upper cone 31 in the assembled nebulizer 1. To this end, the insert part 30 is provided with radially projecting braces or spokes 36 which interconnect the cone top 31a with the stabilizing support cylinder 35. The illustrated embodiment comprises four spokes 36, but the number may vary. As best seen in Figs 3 and 4, the spokes 36 define a number of passage openings 37 of the insert part 30. The passage openings 37 allow liquid filling and aerosol outflow.

The internal tubular part 40 of the nebulizer 1 is shaped as an axially extending cylinder having an outwardly flaring skirt 42 at its axially lower end. The skirt 42 defines a lower opening of an inner space 41. The outer diameter of the tubular part corresponds to the inner diameter D2 of the lower section 14a of the upper wall portion 14 of the housing 10. An outer thread 44 is formed at the upper end of the tubular part 40 for connecting the tubular part 40 to the housing 10 at the inner thread 15. As will be described in detail later, one purpose of the tubular part 40 is to create a liquid trap. Another purpose of the tubular part 40 is to act as a guide for the reverse aerosol flow. In alternative embodiments, the tubular part 40 is integrally formed in one piece with the housing 10, with no threads 44 and 15. In alternative embodiments, the tubular part 40 is dismissed with.

The top part 50 of the nebulizer 1 has a cylindrical wall provided with a radially extending flange 53. Axially below the flange 53, the top part presents a larger wall thickness and an outer thread 52 for connecting the top part 50 to the housing 10 at the inner thread 16. At its axially lower end, the cylindrical wall of the top part 50 presents a circumferentially extending and outwardly flaring skirt 54 defining a ring-shaped cavity 55 on the radially outer side of the top part 50. At its lower end, the top part 50 supports an impact surface termed baffle 60 via a plurality of inwardly inclined braces 61. The number of braces 61 is four in the illustrated embodiment, but may be varied. The braces 61 are inclined to facilitate the aerosol outflow. Inclined openings 62 defined between the braces 61 allow liquid filling and aerosol outflow. The openings 62 have rounded corners to facilitate the aerosol outflow.

The main parts 10, 20, 30, 40, 50 can be assembled in different orders to make the final nebulizer 1. The following is a non-limiting example of an assembly sequence:
As a first assembly step, the top part 50 is connected to the housing 10 at threads 52 and 16. This will position the baffle 60 inside the housing 10 at an axial level which is preferably adjustable, as will be described later. This will also create a liquid trap, termed second liquid trap T2, at the ring-shaped cavity 55, the purpose of which will be described later.
As a second assembly step, the tubular part 40 is inserted through the open lower end of the housing 10, and is attached to the housing 10 at threads 44 and 15. This will create a first liquid trap T1, which is larger than the second liquid trap T2 and the purpose of which will be described later.
As a third assembly step, the insert part 30 is inserted through the lower open end of the housing 10 and is received partly into the tubular part 40. The support cylinder 35 of the insert part 30 is received in the middle section 14b of the upper wall portion 14 of the housing 10. Optionally, a minor press fit may be provided to hold the insert part 30 in place. The press fit may be releasable. The upper cone 31 is now positioned inside the housing 10. Thereby, an inner volume V is created inside the housing 10 between the sidewall of the housing 10 and the concave conical surface 32 of the upper cone 31.
As a fourth and final assembly step, the bottom part 20 is attached to the housing 10 at the threads 21c and 11a. This will position the lower cone 22 inside the upper cone 31. This final assembly step will create an aerosol generating nozzle formed by the two cone tops 22a, 31a, and it will also create a liquid passageway 71 in the conical structure. The final assembly step will also secure the insert part 30 in place inside the housing 10.

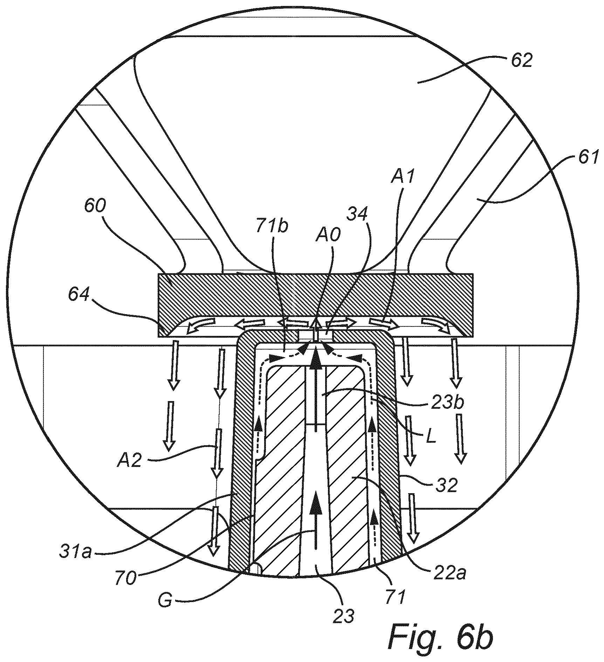

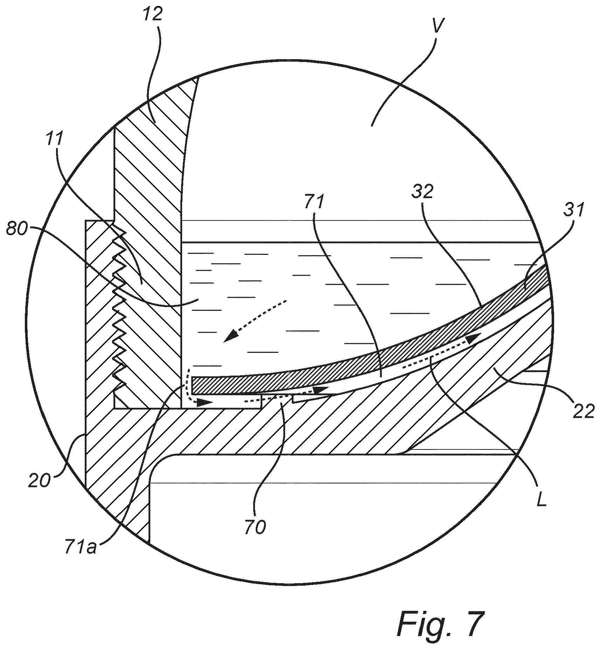

As seen in Figs 1 and 4, and especially in enlarged scale in Fig. 7, the lower cone 22 is provided with a plurality of spacers 70. The purpose of these spacers 70 is to position the lower cone 22 and the upper cone 31 in an exact spaced mutual relation to each other for creating the conical liquid passageway 71 between the lower cone 22 and the upper cone 31. The number of spacers 70, and the circumferential and axial distribution thereof may vary. In the illustrated embodiment, the spacers 70 include a plurality of lower, circumferentially distributed spacers 70 extending upwardly from the lower cone 22 close to the cone base 22b (Fig. 7), and a plurality of upper, circumferentially distributed spacers 70 extending radially outwardly from the lower cone close to the cone top 22a (Fig. 6b). During the final assembly step when the bottom part 20 is connected to the housing 10, the spacers 70 will make contact with the upper cone 31, thereby creating the liquid passageway 71. In alternative embodiments, the spacers 70 may be provided on the upper cone 31 instead, or on both cones 22, 31. The liquid passageway 71 extends from the cone base 31b to the cone top 31a, and extends 360 degrees peripherally with respect to the main axis A. Liquid may pass between the spacers 70. The liquid passageway 71 has a liquid inlet 71a at the cone base 31a, and a liquid outlet 71b at the cone top 31a. The passageway 71 may be a capillary liquid passageway 71. In alternative embodiments, the liquid may be drawn to the cone top 31a by alternative shapes of liquid channels.

In the illustrated embodiment as seen in Fig. 7, the liquid inlet 71a of the liquid passageway is formed by a radial clearance or gap between the cone base 31b of the upper cone 31 and the inside of the base wall portion 11 of the housing 10. As will be discussed later, the liquid inlet 71a is thereby located at a favorable position in the inner volume V with respect to the ability of the nebulizer 1 to draw liquid into the liquid passageway 71 in both the upright orientation and in tilted orientations of the nebulizer 1. It will be noted that the liquid inlet 71a is located at an axial level where the inner volume V presents its largest cross-section, corresponding to the largest diameter D1. In alternative embodiments, the liquid inlet 71a may be formed as a plurality of circumferentially distributed liquid inlets located at or close to the periphery of the cone base 31b, for instance formed by a toothed periphery of the cone base 31b, or formed by small holes in the cone base 31b.

In addition to forming the liquid passageway 71 together with the lower cone 22, a purpose of the conical insert 30 is to provide an effective and novel aerosol flow guiding surface 32 inside the housing 10. The concave conical shape of the flow guiding surface 32 has the advantage of providing a high speed, and controlled aerosol flow down into the inner volume V, and also to create an aerosol vortex formation inside the inner volume V as will be described later. The concave shape also contributes to an enlarged inner volume V.

An advantage obtained by the inventive concept is the provision of an inner volume V the relative dimensions of which is essentially larger than in known nebulizers. This, in its turn, has a positive effect on the aerosol generation performance of the nebulizer 1 as will be discussed more in detail below. The enlarged size of the inner volume V is partly due to the conical surface 32 being concave the inner volume V and, thereby, enlarging the inner volume V, and partly due to design of the sidewall of the housing 10 which has its inwardly inclined portion 13 axially spaced from the lower part of the inner volume V, thereby contributing to a larger inner volume at the lower end of the housing 10.

In the assembled nebulizer in Fig. 1, the tubular part 40 extends down into the inner volume V. The lower flaring end 42 of the tubular part 40 is axially located between the cone top 31a and the cone base 31b. In relation to the sidewall, the flaring end 42 is located at about the same axial level as the transit wall portion 12. Thereby, the first liquid trap T1 is formed as a downwardly open cavity, which is formed between the downwardly projecting tubular part 40 and the inclined wall portion 13, and which extends circumferentially about the axis A. Referring to Fig. 8a, the flaring end 42 of the tubular part 40 is located at an axial distance from a liquid level 81 of the liquid 80 inside the inner volume V, when the nebulizer 1 is in its upright position.

With respect to the axial locations of the sidewall portions 11, 12, 13 of the housing 10, it is noted that the base wall portion 11 is located below the tubular part 40, the transition portion 12 is located about in level with the lower end 42 of the tubular portion 40 and between the cone top 31a and the cone base 31b, and at least the majority of the inclined portion 13 is located above the lower end of the tubular part 40 to form the first liquid trap T1.

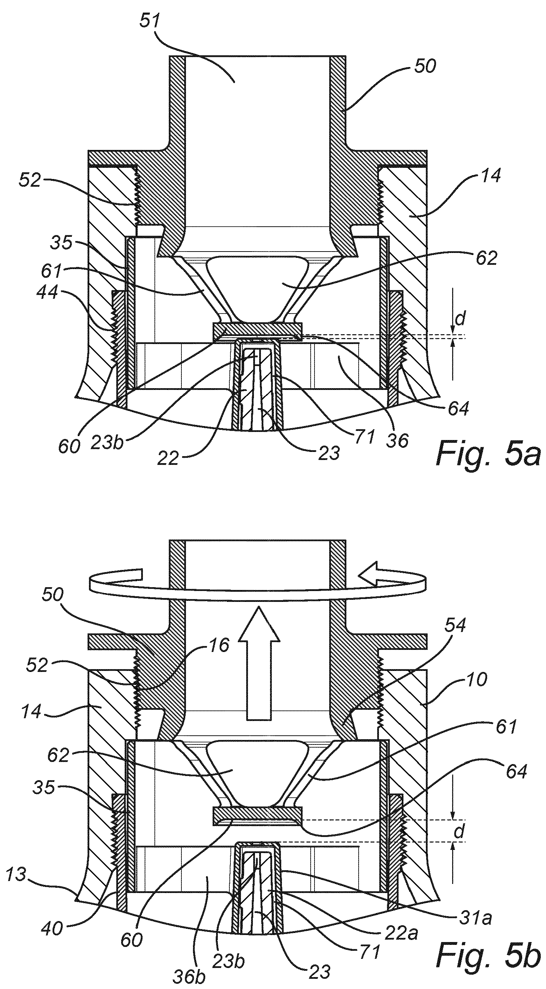

As a result of the assembly steps described above, an aerosol generating structure is created inside the nebulizer 1. In short and as best shown in Fig. 6b, an aerosol generating nozzle is formed by the combination of the two cone tops 22a and 31a, the gas outlet 23b, and the nozzle orifice 34. As shown in Fig. 5a and 5b, this aerosol generating nozzle is located at a distance d from the underside of the baffle 60. The baffle 60 will also take part of the aerosol flow formation as will be described later. The baffle 60 has an aerosol flow guiding structure 64 structured and arranged to guide the aerosol flow along the concave conical surface 32. In the illustrated embodiment, the baffle 60 is in the form of a circular plate, which is supported by the braces 61 and which on its underside has an impact surface arranged to receive a high-speed liquid/gas mixture jet stream from the nozzle.

The operation of the nebulizer 1 will now be described with reference to Figs 5 to 8b. Initially, a predetermined volume or dose of a medical liquid is placed in the nebulizer 1. This may be done by placing the nebulizer 1 in its upright position as shown in Fig. 8a on a table or similar, without any gas hose attached to the hose nipple 24, and without any face mask or similar (not shown) attached to the top part 50. Liquid filling may also be performed by holding the nebulizer with a minor tilt. It is also possible to have the gas hose attached during liquid filling. A predetermined volume of a medical liquid is then poured into the nebulizer 1 through the top part 50 at reference numeral 51. The liquid is collected at the lower part of the inner volume V, acting as a liquid reservoir. The situation is now essentially as shown in Fig. 8a. The dimensions of the nebulizer 1 in relation to the fill volume should be such that the liquid level 81 of the fill volume 80 is axially spaced from the lower end 42 of the tubular part 40. If the nebulizer 1 is intended for re-use, the nebulizer 1 may be cleaned before liquid filling by detaching the various parts from each other.

The fill volume may be varied depending on several factors. One factor is the so-called dead volume. In general, a suitable fill volume for a jet nebulizer depends on the dead volume of the nebulizer. The dead volume refers to the total remaining liquid volume which is not atomized and not administered to the patient. In general, by increasing the fill volume, the proportion of the dead volume is reduced. Other factors may relate to the pressurized gas used for operating the nebulizer. As an example, the fill volume may be less than 10 ml, and typically in the interval of 2-5 ml. A normal prescription dose is 2,5 ml per treatment. It may be noted that the dead volume, for jet nebulizers in general, is formed by liquid present on the inner surfaces of the structure as droplets, plus any liquid remaining in the actual liquid reservoir that for some reason cannot be drawn into the aerosol generating structure. As will be described below, in the inventive nebulizer 1, the non-droplet portion of the dead volume is very low, both for the upright orientation and for a tilted orientation of the nebulizer, since essentially all liquid present in liquid-form (non-droplet form) at the lower part of the inner volume V will be drawn into the fluid inlet 71a. Another factor regarding the fill volume relates to the ability of the inventive nebulizer to hold the liquid inside the nebulizer in all possible orientations, even in upside down inverted orientations. The liquid fill volume should therefore preferably be adapted to the total size of the two liquid traps T1 and T2. In some embodiments, the liquid fill volume is such that the first liquid trap T1 alone can hold the entire fill volume. Still another factor determining a suitable fill volume is that the fill volume should not be so large that it negatively influences the aerosol flow in the inner volume, especially the vortex formation and the reverse flow.

Next, a source of pressurized gas (not shown) is connected to the gas inlet 23a of the gas channel 23 via the hose nipple 24. With the nebulizer 1 now ready for use, the nebulizer outlet 51 of the top part 50 is typically connected to a patient interface, such as a face mask or a mouthpiece (not shown).

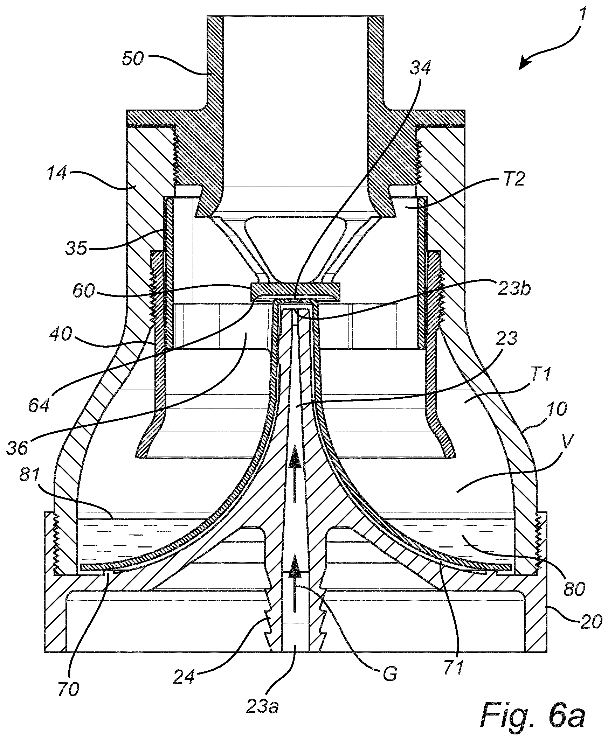

The gas source is now turned on, creating a gas flow G in the gas channel 23 as illustrated in Fig. 6a. The gas volume flow rate at the gas inlet 23a may as an example be in the order of 6-10 I/min. The gas flow rate may influence the particle size in the aerosol, and the impact distance d at the baffle 60 (Figs 5a and 5b) will also influence on the particle size. However, the gas flow rate has a high influence on the nebulizer output, i.e. the total volume of aerosol mist discharged from the nebulizer 1. The gas flow rate in the gas channel 23 may be very high, and increases towards the gas outlet 23b due to the tapered design of the gas channel 23. In preferred embodiments, the gas flow rate at the gas outlet 23b may be around 330 m/s. Deliberately, the gas flow speed is maintained in this region, since speed closer to the speed of sound may entail the known phenomenon of greatly increased resistance, as well as subsequent mechanical stress and other disadvantages of the so-called "sonic bang". However, the still high speed of about 330 m/s has the advantage of giving a strong impact effect against the baffle 60, and thus a good amount and quality of the nebulization mist.

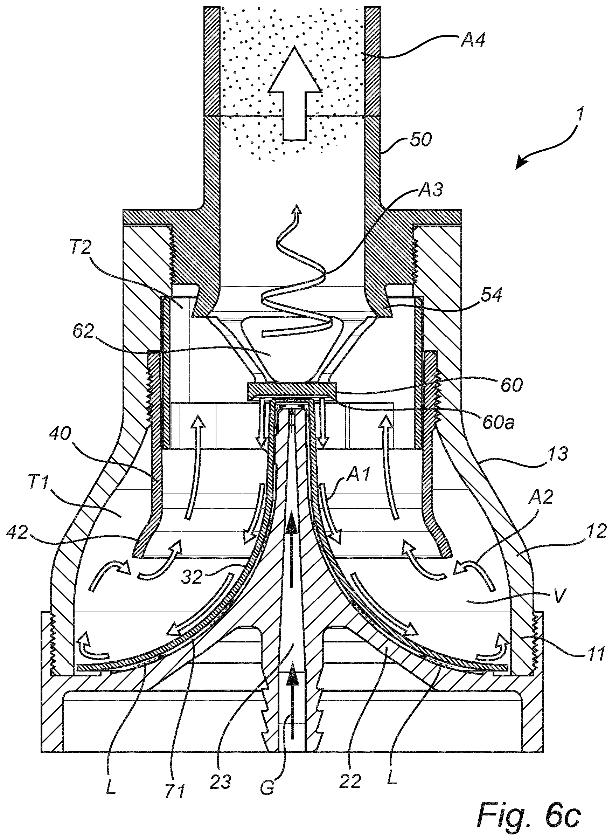

Reference is now made to Figs 6b, 6c, and 7. In response to the high-speed gas flow G exiting the gas outlet 23b of the lower cone 22, a region of negative pressure is created just above the gas outlet 23b. In response to the negative pressure and venturi effect, the liquid 80 present in the inner volume V is drawn into the liquid inlet 71a (Fig. 7), through the liquid passageway 71 as a liquid flow L (Fig. 6b and Fig. 7), and up to the liquid outlet 71b. Just above the gas outlet 23b, the liquid flow L meets the gas flow G. Thereby, a gas/liquid mixture jet stream is created as shown by an upwardly directed white arrow A0 in Fig. 6b. This is the first location where an aerosol mist is created, but with a relatively larger particle size.

The gas/mixture jet stream A0 exits through the nozzle orifice 34 of the upper cone 31, and impacts with great force on the impact surface of the baffle 60. The main purpose of the baffle 60 is to increase turbulence and particle collisions, which reduces the particle size. Thereby, an initial aerosol flow A1 is created having a more optimal droplet or particle size distribution. As schematically shown by white arrows in Fig. 6b, the initial aerosol flow A1 is directed radially outwards from the axis A below the baffle 60.

A minor part of the initial aerosol flow A1 may go directly upwards past the edges of the baffle and out into the "chimney" and into the patient's mask. At gas inflow rates of 6-10 I/min or more, this "direct" upward outflow may contribute to a vortex moving upward in the "chimney" as illustrated at reference A3 in Fig. 6c, reducing particle size through particle collisions in the vortex, as well as increasing the output flow.

The shape and dimensions of the baffle 60 may be optimized with respect to two factors: on the one hand, the area of the impact surface of the baffle 60 should be large enough to create a sufficient aerosol mist. On the other hand, the baffle 60 should be designed to allow the created reverse aerosol flow to exit pass the baffle 60. The vibration stability of the baffle 60 is also a relevant factor. The illustrated design provides a stable vibration resistant baffle 60. Avoiding baffle vibration contributes to an optimized particle size distribution, and an optimized aerosol flow.

A majority of the initial aerosol flow A1 flowing radially outwardly below the baffle 60 is directed downwardly by the flow guiding structure 64 of the baffle 60. Thus, a majority of the initial aerosol flow A1 is directed downwardly towards and along the concave conical surface 32. The flow guiding structure 64 is in this embodiment formed by a circumferentially and downwardly extending edge or skirt having an inclined radially inner impact surface for guiding the aerosol flow A1. In alternative embodiments, the flow guiding may be implemented by means separate from the baffle.

The initial aerosol flow A1 is now a mixed flow, with a laminar slower part closest to the concave cone surface 32, peripherally followed by a laminar/turbulent boundary layer, and in an even more peripheral part of the downflow, a turbulent flow, which, however, is still directed downwards in its total direction (mean flow).

As schematically shown in Fig. 6c, at the lower part of the inner volume V the direction of the downward aerosol flow A1 is reversed to form an upwardly directed reverse aerosol flow A2. The flow direction is reversed partly due to the shape of the structure, especially the formation of the relatively large space in the lower part of the inner volume V and the presence of the lower wall portion 11, and partly due to a vortex formation occurring in the lower peripheral part of the inner volume V. The flow reversal is also influenced by the liquid surface. The reverse flow is of mixed type as for the downward flow A1, i.e. largely turbulent. This reverse "flushing" technique according to the inventive concept generates a stable and high output from the nebulizer. It also contribute to a high degree of particle collisions, resulting in a larger amount of smaller or relatively small particles in the resulting aerosol flow.

At the transition between downward flow A1 and upward reverse flow A2, a vortex is formed in the inner volume V by these two flows in combination. The vortex is of the torus vortex type. This means that it has a tubular closed circular shape, much like a "ring doughnut". The rotational motion of the vortex is maintained by the downward aerosol flow A1. The rotation of the vortex contributes to the direction and the speed of the upward reverse aerosol flow A2, which in turn provides a good outflow A4 from the nebulizer 1. The rotation of the torus vortex also contributes to an increased degree of particle collisions, resulting in a reduced particle size. The vortex formation occurs already at 6 I/min, and increases with an increased gas flow. Many prior-art nebulizers require a gas inflow rate of 8 I/min to operate, whereas the inventive nebulizer may be implemented to operate at 6 I/min. This is more advantageous if, for example, there is a need to treat patients with chronic lung disease using oxygen, where too much oxygen can have negative effects to the patient. Obtaining a vortex already at 6 I/min is very good. The fact that the vortex increases with the increased flow is also very good. The reverse aerosol flow A2 contributes to the vortex moving upward in the "chimney" as illustrated at reference A3 in Fig. 6c.

Reference is now made to Figs 5a and 5b. As described above, the top part 50 is connected to the housing 10 at threads 16 and 52. Thereby, the axial position of the top part 50 in relation to the housing 10 can be adjustable by turning the top part 50 as indicated in Fig. 5b. Since the baffle 60 is a part of the top part 50, the baffle or impact distance d is thus adjustable. For illustration purposes, Fig. 5b illustrates in a very excessively manner how the impact distance d can be enlarged. In real life, the adjustment range is much smaller, for instance an interval from 0,2 mm to 1,0 mm. An adjustable impact distance d has the advantage that a suitable particle size distribution can be preset, so that a suitable aerosol can be formed for a given gas flow rate. This makes it possible to achieve an optimum treatment of various disease states in the lungs. By setting the dominant part of the particle size, it is possible to efficiently and accurately reach various parts of the lungs, which is an important factor in order to get the best treatment effect in different disease states

The rotation of the top part may be combined with tactile means and/or visual indicators for setting of the impact distance. The adjustment may be continuous or stepwise, with specific settings for various treatments. As an alternative to making the distance d adjustable as shown in Figs 5a and 5b, the nebulizer may be delivered with a set of different top parts 50 having different fixed distances d. Each of these parts may optionally also be adjustable as shown in Figs 5a and 5b.

Reference is now made to Figs 8a and 8b, illustrating a liquid level inside the inventive nebulizer 1 in its upright orientation, and in a tilted horizontal orientation, respectively. In Fig. 8A, the nebulizer 1 is in its vertical upright position. A liquid has been introduced through the top part 50 and is present at the lower part of the inner volume V. The dimensions of the nebulizer1 and the fill volume of the liquid 80 are such that the liquid level 81 is below the lower end 42 of the tubular structure 40, and also below the first liquid trap T1. In the illustrated example, the liquid level 81 of the fill volume is about in axial level with the top of the base wall portion 11.

As shown in enlarged scale in Fig. 7, the liquid inlet 71a is located where the liquid 80 has its deepest point, namely at the outer periphery of the cone base 31b. This will ensure that essentially all liquid present in the inner volume V will be drawn into the liquid channel 71 and aerosolized. Only a minor residual part of the original fill volume may eventually remain on the inner surface of the structure. This residual part can be minimized by tapping as known in the art. During operation in the upright orientation in Fig. 8a, the aerosol flow A1 and A2 as described above, especially including the flow reversal and the vortex formation, will take place inside the inner volume V. The flow reversal may take part at the surface level 81 and, accordingly, will occur at a gradually lower level as the liquid is consumed.

In Fig. 8b, the nebulizer 1 in Fig. 8a has been tilted to a horizontal orientation. In the illustrated example, the fill volume of the liquid 84 is such that the liquid level 85 is somewhat above the lower part of the tubular structure 40. Part of the liquid is present in the first liquid trap T1, and a small part of the liquid is present in the smaller second liquid trap T2. It will be appreciated that the lower part of the inclined wall portion 13 in Fig. 8b will, per se, function as a liquid barrier preventing the liquid from leaving the nebulizer 1 in tilted orientations, and acting as a downhill slope guiding the liquid 84 towards the liquid inlet 71a. However, for larger fill volumes that cannot be handled by the inclined wall part 13 alone, it is preferred to provide at least the first liquid trap T1 and, in preferred embodiments, also an additional second liquid trap T2 located at an axial higher level, above the baffle 60.

Disregarding the liquid traps T1 and T2, which are both optional, it will be appreciated that the base wall portion 11 and the inclined wall portion 13 together form a liquid tray in a tilted horizontal orientation as illustrated in Fig. 8b, efficiently collecting the liquid 84 such that it may be drawn in at the liquid inlet 71a.

The inventive nebulizer 1 operates not only almost as efficient in tilted orientations as in the upright position. The inventors have demonstrated that the inventive nebulizer 1 operates equally efficient in the horizontal orientation as in the upright orientation. This is a major advantage compared to present jet nebulizers. In the tilted orientation, the aerosol generating structure (nozzle and baffle 60) are still located above the liquid level 85, and the generated aerosol flow A1 will also in this horizontal orientation be guided along the concave conical surface 32 and be redirected into the reverse flow A2, including the vortex formation, with all the advantages related thereto as described above. Also, in the tilted orientation, the liquid inlet 71a has at least one part located where the liquid 84 has its deepest or lowest point, i.e. at the bottom of the base wall portion 11. This ensures that essentially all of the liquid present in the inner volume (excluding liquid present on the inner sidewalls) can be drawn into the liquid passage 71 and transformed into an aerosol flow.

With respect to the two liquid traps T1 and T2, the inventors have found that if the size of the first liquid trap T1 is increased too much (for handling a larger amount of liquid in tilted orientations), this may have a negative impact on the reverse flow and the vortex formation. For this reason, it may be preferred to include also the second liquid trap T2 axially higher up, at a position where the second liquid trap T2 does not disturb the aerosol flow. In alternative embodiments, there is only one liquid trap T1. In even simpler embodiments there is no liquid trap, whereby the inclined wall portion 13 will alone have to act as a liquid barrier for preventing liquid outflow in the tilted orientation.

The two liquid traps T1 and T2 (or in some alternatives a single liquid trap T1) may preferably be dimensions such that, even if the nebulizer 1 in Fig. 8b for some reason is inverted completely, i.e. oriented upside down, then essentially all the liquid would be retained by the liquid trap(s). As an example, the two liquid traps T1 and T2 are preferably dimensioned to handle at least a fill volume of 5 ml. In an inverted orientation, obviously no liquid would be present at the liquid inlet 71a and no aerosol generated, but since no liquid is spilled out, the aerosol generation will continue as soon as the nebulizer 1 is turned back to an operational orientation.

### Second inventive concept

According to a second inventive concept, which is intended to be subject of a potential divisional application, there is provided a jet nebulizer comprising:
a housing;
a nozzle which supported inside the housing and arranged to generate a liquid/gas mixture from a liquid and a compressed gas; and
a top part which supports a baffle at a distance from the nozzle, said baffle having an impact surface arranged to receive the liquid/gas mixture from the nozzle to form an aerosol flow,
wherein the top part is adjustably attached to the housing for allowing said distance between the nozzle and the baffle to be adjusted.

This second inventive concept of creating an adjustable baffle distance inside a nebulizer should be considered as an inventive concept separate from the first inventive concept described above, directed to the concave conical flow guiding surface, and may be the subject-matter of a divisional application in which features that are essential features in the present invention are optional in the second inventive concept. Thus, the second inventive concept may be implemented in a nebulizer with or without a concave conical surface as described above, and with or without the inclined design of the sidewall as described above.

However, in preferred embodiments of the second inventive concept, the structure and operation of the nebulizer is in accordance with the embodiment described above with reference to the figures. In such preferred embodiments, the above description and examples apply.

In a preferred embodiment of the second inventive concept, the top part of the nebulizer is adjustably attached to the housing by a screw connection adjustable by a user. The top part and the housing may be provided with matching screw threads, extending circumferentially with respect to a main axis of the nebulizer. By manually rotating the top part in relation to the housing, a user can adjust the nozzle-baffle distance (impact distance) along the main axis. The adjustment can be continuous or stepwise, and may be provided with a tactile feedback structure defining different rotational positions.

The adjustability of the nozzle-baffle distance has the advantage that the properties of the created aerosol flow can be optimized depending on various parameters, such as a desired droplet size distribution, viscosity of the liquid being aerosolized, the flow rate of the compressed gas, and the speed of the aerosol jet stream impacting on the baffle.

## Claims

1. A jet nebulizer (1) for creating an aerosol from a liquid, such as a liquid medication, comprising:
a tubular housing (10) having a sidewall which extends circumferentially about a main axis (A) of the nebulizer;
wherein a conical structure having a concave conical surface (32) , which extends between a cone top (22a, 31a) and a cone base (22b, 31b), which together with the sidewall of the tubular housing forms an inner volume (V) in the nebulizer; and
an aerosol generating structure which is arranged to generate an initial aerosol flow from said liquid and to guide the initial aerosol flow along the concave conical surface (32) towards the cone base,
wherein a base wall portion (11) of the sidewall extends from an axial level of the cone base (22b, 31b), with respect to the main axis, to an axial level between the cone top (22a, 31a) and the cone base (22b, 31b) where the base wall portion (11) transits into an inclined wall portion (13) of the sidewall, said inclined wall portion being inclined in relation to the base wall portion inwardly towards said main axis; and
wherein the sidewall is arranged to redirect the initial aerosol flow guided along the concave conical surface to form a reverse aerosol flow in the inner volume.

2. The nebulizer (1) according to claim 1, wherein the sidewall further comprises a curved wall portion (12), which forms a curved transition of the sidewall between the base wall portion and the inclined wall portion, said curved wall portion presenting a concave inner surface facing the inner volume.

3. The nebulizer (1) according to claim 1 or 2, wherein the inner volume (V) presents its largest cross section, with respect to said main axis (A), at the cone base(22b, 31b).

4. The nebulizer (1) according to any of claims 1 to 3, wherein the conical structure comprises a central compressed air passageway, and a liquid passageway which is arranged peripherally of the compressed air passageway and which has a liquid inlet located at the sidewall of the tubular housing.

5. The nebulizer (1) according to any of the preceding claims, further comprising an internal tubular structure which projects into the inner volume and which has an opening located at an axial level, with respect to the main axis, between the cone top and the cone base, wherein said internal tubular structure together with a portion of the sidewall forms a cavity arranged to act as a liquid trap for the liquid in a tilted orientation of the nebulizer.

6. The nebulizer (1) according to claim 5, wherein said opening of the internal tubular structure is formed by an outwardly flaring skirt portion of the internal tubular structure.

7. The nebulizer (1) according to any of the preceding claims, wherein the aerosol generating structure comprises a baffle provided with a guide for guiding the generated initial aerosol towards the concave conical surface.

8. The nebulizer (1) according to any of the preceding claims, wherein the aerosol generating structure comprises a baffle provided with an impact surface, and a nozzle, which is spaced at a distance from the impact surface of the baffle arranged to direct a liquid/gas mixture towards the impact surface, and wherein said distance is adjustable.

9. A method for generating an aerosol flow in a jet nebulizer, said method comprising:
generating an initial aerosol inside a nebulizer from a liquid and a pressurized gas;
guiding the initial aerosol as a primary aerosol flow along a concave conical surface from a top of the concave conical surface towards a base of the concave conical surface, said concave conical surface together with a sidewall of the nebulizer defining an inner volume of the nebulizer;
at the base of the concave conical surface, redirecting the aerosol flow by the sidewall to form a reverse aerosol flow; and
guiding the reverse aerosol through the inner volume to an outlet of the nebulizer.

10. The method according to claim 9, further comprising using said primary aerosol flow and said reverse aerosol flow in combination to form an aerosol vortex within said inner volume.

11. The method according to claim 9 or 10,
wherein said action of generating an initial aerosol comprises forming an aerosol stream by a nozzle of the nebulizer, and impacting the aerosol stream onto a baffle of the nebulizer for forming the initial aerosol; and
wherein said action of guiding the initial aerosol along the concave conical surface comprises guiding the initial aerosol by a flow guide structure, which is provided on the baffle and is arranged to direct the initial aerosol towards the concave conical surface.

12. The method according to any of claims 9 to 11, wherein the action of redirecting the aerosol flow at the base of the concave conical surface comprises redirecting the primary aerosol flow by a base wall portion of the sidewall, which base wall portion transits, at a level between the top and the base of the concave conical surface, into an inclined wall portion, which is inclined in relation to the base wall portion inwardly towards a main axis of the nebulizer.

13. The method according to any of claims 9 to 12, wherein the action of guiding the reverse aerosol flow towards the outlet of the nebulizer further comprises guiding the reverse aerosol flow from the sidewall and into an internal tubular structure which projects into the inner volume.

14. The method according to claim 13, wherein the internal tubular structure has an opening for receiving the reverse aerosol flow located at an axial level, with respect to said main axis, between a top and the base of the concave conical surface.

15. The method according to claim 14, wherein the opening of the internal tubular structure is formed, with respect to said main axis, by a radially outwardly flaring skirt portion of the tubular structure.

16. The method according to any of claims 9 to 15, wherein the action of generating the initial aerosol comprises drawing the liquid, in response to application of the pressurized gas, from said inner volume into a liquid inlet formed by a clearance between the base of the concave conical surface and the sidewall, and through a conical liquid passageway below the concave conical surface.

## Patentansprüche

1. Strahlvernebler (1) zum Erzeugen eines Aerosols aus einer Flüssigkeit, wie beispielsweise einem flüssigen Arzneimittel, der Folgendes umfasst:
ein röhrenförmiges Gehäuse (10), das eine Seitenwand aufweist, die sich umlaufend um eine Hauptachse (A) des Verneblers erstreckt,
wobei
eine kegelförmige Struktur eine konkave kegelförmige Oberfläche (32) aufweist, die sich zwischen einer Kegelspitze (22a, 31a) und einer Kegelbasis (22b, 31b) erstreckt, die zusammen mit der Seitenwand des röhrenförmigen Gehäuses ein inneres Volumen (V) in dem Vernebler bildet, und
eine Aerosol erzeugende Struktur, die dafür angeordnet ist, einen anfänglichen Aerosolstrom aus der Flüssigkeit zu erzeugen und den anfänglichen Aerosolstrom entlang der konkaven kegelförmigen Oberfläche (32) hin zu der Kegelbasis zu leiten,
wobei sich ein Basiswandabschnitt (11) der Seitenwand von einem axialen Niveau der Kegelbasis (22b, 31b), in Bezug auf die Hauptachse, bis zu einem axialen Niveau zwischen der Kegelspitze (22a, 31a) und der Kegelbasis (22b, 31b) erstreckt, wobei der Basiswandabschnitt (11) in einen geneigten Wandabschnitt (13) der Seitenwand übergeht, wobei der geneigte Wandabschnitt im Verhältnis zu dem Basiswandabschnitt nach innen hin zu der Hauptachse geneigt ist, und
wobei angeordnet ist, um den anfänglichen Aerosolstrom, der entlang der konkaven kegelförmigen Oberfläche geleitet wird, umzulenken, um einen umgekehrten Aerosolstrom in dem inneren Volumen zu bilden.

2. Vernebler (1) nach Anspruch 1, wobei die Seitenwand ferner einen gekrümmten Wandabschnitt (12) umfasst, der einen gekrümmten Übergang der Seitenwand zwischen dem Basiswandabschnitt und dem geneigten Wandabschnitt bildet, wobei der gekrümmte Wandabschnitt eine kegelförmige Innenfläche aufweist, die zu dem inneren Volumen zeigt.

3. Vernebler (1) nach Anspruch 1 oder 2, wobei das innere Volumen (V) seinen größten Querschnitt, in Bezug auf die Hauptachse (A), an der Kegelbasis (22b, 31b) aufweist.

4. Vernebler (1) nach einem der Ansprüche 1 bis 3, wobei die kegelförmige Struktur einen mittigen Pressluftdurchgang und einen Flüssigkeitsdurchgang, der peripher von dem Pressluftdurchgang angeordnet ist und der einen Flüssigkeitseinlass aufweist, der an der Seitenwand des röhrenförmigen Gehäuses angeordnet ist, umfasst.

5. Vernebler (1) nach einem der vorhergehenden Ansprüche, der ferner eine innere röhrenförmige Struktur umfasst, die in das innere Volumen vorspringt und die eine Öffnung aufweist, die bei einem axialen Niveau, in Bezug auf die Hauptachse, zwischen der Kegelspitze und der Kegelbasis angeordnet ist, wobei die innere röhrenförmige Struktur zusammen mit einem Abschnitt der Seitenwand einen Hohlraum bildet, der angeordnet ist, um bei einer geneigten Ausrichtung des Verneblers als eine Flüssigkeitsfalle für die Flüssigkeit zu wirken.

6. Vernebler (1) nach Anspruch 5, wobei die Öffnung der inneren röhrenförmigen Struktur durch einen sich nach außen aufweitenden Schürzenabschnitt der inneren röhrenförmigen Struktur gebildet wird.

7. Vernebler (1) nach einem der vorhergehenden Ansprüche, wobei die Aerosol erzeugende Struktur eine Ablenkplatte, die mit einer Führung zum Leiten des erzeugten anfänglichen Aerosolstroms hin zu der konkaven kegelförmigen Oberfläche versehen ist, umfasst.

8. Vernebler (1) nach einem der vorhergehenden Ansprüche, wobei die Aerosol erzeugende Struktur eine Ablenkplatte, die mit einer Aufprallfläche versehen ist, und eine Düse, die in einer Entfernung von der Aufprallfläche der Ablenkplatte beabstandet ist, wobei sie angeordnet ist, um ein Flüssigkeit-Gas-Gemisch hin zu der Aufprallfläche zu richten, umfasst und wobei die Entfernung einstellbar ist.

9. Verfahren zum Erzeugen eines Aerosolstroms in einem Strahlvernebler, wobei das Verfahren Folgendes umfasst:
Erzeugen eines anfänglichen Aerosolstroms innerhalb eines Verneblers aus einer Flüssigkeit und einem unter Druck gesetzten Gas,
Führen des anfänglichen Aerosolstroms als einen primären Aerosolstrom entlang einer konkaven kegelförmigen Oberfläche von einer Spitze der konkaven kegelförmigen Oberfläche hin zu einer Basis der konkaven kegelförmigen Oberfläche, wobei die konkave kegelförmige Oberfläche zusammen mit einer Seitenwand des Verneblers ein inneres Volumen des Verneblers definiert,
an der Basis der konkaven kegelförmigen Oberfläche Umlenken des Aerosolstroms durch die Seitenwand, um einen umgekehrten Aerosolstrom zu bilden, und
Führen des umgekehrten Aerosolstroms durch das innere Volumen zu einem Auslass des Verneblers.

10. Verfahren nach Anspruch 9, das ferner das Verwenden des primären Aerosolstroms und des umgekehrten Aerosolstroms in Kombination umfasst, um einen Aerosolwirbel innerhalb des inneren Volumens zu bilden.

11. Verfahren nach Anspruch 9 oder 10,
wobei der Vorgang des Erzeugens eines anfänglichen Aerosolstroms das Bilden eines Aerosolstroms durch eine Düse des Verneblers und das Aufprallenlassen des Aerosolstroms auf eine Ablenkplatte des Verneblers zum Bilden des anfänglichen Aerosolstroms umfasst und
wobei der Vorgang des Führens des anfänglichen Aerosolstroms entlang der konkaven kegelförmigen Oberfläche das Führen des anfänglichen Aerosolstroms durch eine Strömungsführungsstruktur umfasst, die an der Ablenkplatte bereitgestellt wird, und angeordnet ist, um den anfänglichen Aerosolstrom hin zu der konkaven kegelförmigen Oberfläche zu leiten.

12. Verfahren nach einem der Ansprüche 9 bis 11, wobei der Vorgang des Umlenkens des Aerosolstroms an der Basis der konkaven kegelförmigen Oberfläche das Umlenken des primären Aerosolstroms durch einen Basiswandabschnitt der Seitenwand umfasst, wobei der Basiswandabschnitt, bei einem Niveau zwischen der Spitze und der Basis der konkaven kegelförmigen Oberfläche, in einen geneigten Wandabschnitt übergeht, der im Verhältnis zu dem Basiswandabschnitt nach innen hin zu einer Hauptachse des Verneblers geneigt ist.

13. Verfahren nach einem der Ansprüche 9 bis 12, wobei der Vorgang des Führens des umgekehrten Aerosolstroms hin zu dem Auslass des Verneblers ferner das Führen des umgekehrten Aerosolstroms von der Seitenwand und in eine innere röhrenförmige Struktur, die in das innere Volumen vorspringt, umfasst.

14. Verfahren nach Anspruch 13, wobei die innere röhrenförmige Struktur eine Öffnung zum Aufnehmen des umgekehrten Aerosolstroms aufweist, die bei einem axialen Niveau, in Bezug auf die Hauptachse, zwischen einer Spitze und der Basis der konkaven kegelförmigen Oberfläche angeordnet ist.

15. Verfahren nach Anspruch 14, wobei die Öffnung der inneren röhrenförmigen Struktur, in Bezug auf die Hauptachse, durch einen sich in Radialrichtung nach außen aufweitenden Schürzenabschnitt der röhrenförmigen Struktur gebildet wird.

16. Verfahren nach einem der Ansprüche 9 bis 15, wobei der Vorgang des Erzeugens des anfänglichen Aerosolstroms das Ziehen der Flüssigkeit, als Reaktion auf eine Anwendung des unter Druck gesetzten Gases, aus dem inneren Volumen in einen Flüssigkeitseinlass, der durch einen Freiraum zwischen der Basis der konkaven kegelförmigen Oberfläche und der Seitenwand gebildet wird, und durch einen kegelförmigen Flüssigkeitsdurchgang unterhalb der konkaven kegelförmigen Oberfläche umfasst.

## Revendications

1. Nébuliseur à jet (1) destiné à créer un aérosol à partir d'un liquide, tel qu'un médicament liquide, comprenant :
un boîtier tubulaire (10) comportant une paroi latérale s'étendant circonférentiellement autour d'un axe principal (A) du nébuliseur ;
dans lequel
une structure conique comportant une surface conique concave (32), laquelle s'étend entre un sommet de cône (22a, 31a) et une base de cône (22b, 31b) formant, ensemble avec la paroi latérale du boîtier tubulaire, un volume intérieur (V) dans le nébuliseur ; et
une structure de génération d'aérosol conçue pour générer un flux d'aérosol initial à partir dudit liquide et pour guider le flux d'aérosol initial le long de la surface conique concave (32) vers la base du cône,
dans lequel une partie de paroi de base (11) de la paroi latérale s'étend à partir d'un niveau axial de la base de cône (22b, 31b), par rapport à l'axe principal, vers un niveau axial entre le sommet de cône (22a, 31a) et la base de cône (22b, 31b) où la partie de paroi de base (11) transite vers une partie de paroi inclinée (13) de la paroi latérale, ladite partie de paroi inclinée étant inclinée par rapport à la partie de paroi de base vers l'intérieur vers ledit axe principal ; et
dans lequel la paroi latérale est conçue pour rediriger le flux d'aérosol initial guidé le long de la surface conique concave pour former un flux d'aérosol inverse dans le volume intérieur.

2. Nébuliseur (1) selon la revendication 1, dans lequel la paroi latérale comprend en outre une partie de paroi courbée (12) formant une transition courbée de la paroi latérale entre la partie de paroi de base et la partie de paroi inclinée, ladite partie de paroi courbée présentant une surface intérieure concave tournée vers le volume intérieur.

3. Nébuliseur (1) selon la revendication 1 ou 2, dans lequel le volume intérieur (V) présente sa section transversale la plus grande, par rapport audit axe principal (A), au niveau de la base de cône (22b, 31b).

4. Nébuliseur (1) selon l'une quelconque des revendications 1 à 3, dans lequel la structure conique comprend un passage d'air comprimé central, et un passage de liquide agencé à la périphérie du passage d'air comprimé et comportant une entrée de liquide située au niveau de la paroi latérale du boîtier tubulaire.

5. Nébuliseur (1) selon l'une quelconque des revendications précédentes, comprenant en outre une structure tubulaire intérieure, laquelle fait saillie dans le volume intérieur et comporte une ouverture située à un niveau axial, par rapport à l'axe principal, entre le sommet de cône et la base de cône, dans lequel ladite structure tubulaire intérieure forme, ensemble avec une partie de la paroi latérale, une cavité conçue pour agir comme un piège à liquide pour le liquide dans une orientation penchée du nébuliseur.

6. Nébuliseur (1) selon la revendication 5, dans lequel ladite ouverture de la structure tubulaire intérieure est formée par une partie de jupe évasée vers l'extérieur de la structure tubulaire intérieure.

7. Nébuliseur (1) selon l'une quelconque des revendications précédentes, dans lequel la structure de génération d'aérosol comprend un déflecteur doté d'un guide destiné à guider l'aérosol initial généré vers la surface conique concave.

8. Nébuliseur (1) selon l'une quelconque des revendications précédentes, dans lequel la structure de génération d'aérosol comprend un déflecteur doté d'une surface d'impact, et une buse, laquelle est espacée à une distance de la surface d'impact du déflecteur conçu pour diriger un mélange liquide/gaz vers la surface d'impact et dans lequel ladite distance est réglable.

9. Procédé de génération d'un flux d'aérosol dans un nébuliseur à jet, ledit procédé comprenant :
la génération d'un aérosol initial dans un nébuliseur à partir d'un liquide et d'un gaz sous pression ;
le guidage de l'aérosol initial en tant que flux d'aérosol primaire le long d'une surface conique concave à partir d'une partie supérieure de la surface conique concave vers une base de la surface conique concave, ladite surface conique concave définissant, ensemble avec une paroi latérale du nébuliseur, un volume intérieur du nébuliseur ;
au niveau de la base de la surface conique concave, la redirection du flux d'aérosol par la paroi latérale pour former un flux d'aérosol inverse ; et
le guidage de l'aérosol inverse à travers le volume intérieur vers une sortie du nébuliseur.

10. Procédé selon la revendication 9, comprenant en outre l'utilisation dudit flux d'aérosol primaire et dudit flux d'aérosol inverse en combinaison pour former un tourbillon d'aérosol dans ledit volume intérieur.

11. Procédé selon la revendication 9 ou 10,
dans lequel ladite génération d'un aérosol initial comprend la formation d'un écoulement d'aérosol par une buse du nébuliseur, et l'impact de l'écoulement d'aérosol sur un déflecteur du nébuliseur pour la formation de l'aérosol initial ; et
dans lequel ledit guidage de l'aérosol initial le long de la surface conique concave comprend le guidage de l'aérosol initial par une structure de guidage de flux, laquelle est prévue sur le déflecteur et conçue pour diriger l'aérosol initial vers la surface conique concave.

12. Procédé selon l'une quelconque des revendications 9 à 11, dans lequel la redirection du flux d'aérosol au niveau de la base de la surface conique concave comprend la redirection du flux d'aérosol primaire par une partie de paroi de base de la paroi latérale, ladite partie de paroi de base transitant, à un niveau entre la partie supérieure et la base de la surface conique concave, vers une partie de paroi inclinée, laquelle est inclinée par rapport à la partie de paroi de base vers l'intérieur vers un axe principal du nébuliseur.

13. Procédé selon l'une quelconque des revendications 9 à 12, dans lequel le guidage du flux d'aérosol inverse vers la sortie du nébuliseur comprend en outre le guidage du flux d'aérosol inverse à partir de la paroi latérale et dans une structure tubulaire intérieure faisant saillie dans le volume intérieur.

14. Procédé selon la revendication 13, dans lequel la structure tubulaire intérieure comporte une ouverture destinée à recevoir le flux d'aérosol inverse située à un niveau axial, par rapport audit axe principal, entre une partie supérieure et la base de la surface conique concave.

15. Procédé selon la revendication 14, dans lequel l'ouverture de la structure tubulaire intérieure est formée, par rapport audit axe principal, par une partie de jupe évasée radialement vers l'extérieur de la structure tubulaire.

16. Procédé selon l'une quelconque des revendications 9 à 15, dans lequel la génération de l'aérosol initial comprend l'aspiration du liquide, en réponse à l'application du gaz sous pression, à partir dudit volume intérieur vers une entrée de liquide formée par un espace entre la base de la surface conique concave et la paroi latérale, et à travers un passage de liquide conique sous la surface conique concave.
